# FASCICULE DE BREVET EUROPEEN

(11) **EP 1 641 758 B1**
(45) Date de publication et mention de la délivrance du brevet: **29.10.2008**
(21) Numéro de dépôt: 04767437.9
(22) Date de dépôt: 24.06.2004
(51) Int. Cl.: C07D 213/55, C07D 213/56, C07D 413/12, C07D 401/12, C07D 401/06, A61K 31/4418

(54) **DERIVES DE DIPHENYLPYRIDINE, LEUR PREPARATION ET LEUR APPLICATION THERAPEUTIQUE**
DIPHENYLPYRIDINDERIVATE, DEREN HERSTELLUNG UND DEREN THERAPEUTISCHE ANWENDUNG
DIPHENYLPYRIDINE DERIVATIVES, PREPARATION AND THERAPEUTIC APPLICATION THEREOF

(30) Priorité: 26.06.2003 FR 0307757
(43) Date de publication de la demande: 05.04.2006
(73) Titulaire: Sanofi-Aventis, 75013 Paris (FR)
(72) Inventeur: BARTH, Francis, 34680 SAINT GEORGES D`ORQUES (FR); HORTALA, Laurent, 34080 MONTPELLIER (FR); RINALDI-CARMONA, Murielle, F-34680 Saint Georges d'Orques (FR)
(74) Mandataire: Kugel, Dominique
(86) Numéro de dépôt international: PCT/FR2004/001581
(87) Numéro de publication internationale: WO 2005/000817

(56) Documents cités:
- WO-A-97/19063
- WO-A-03/082191
- WO-A-03/084930
- WO-A-03/084943
- FR-A- 2 816 938
- PERTWEE G R: "Cannabinoid receptor ligands: clinical and neuropharmacological considerations relevant to future drug discovery and development" CURRENT OPINION IN INVESTIGATIONAL DRUGS, CURRENT DRUGS, LONDON, GB, vol. 9, no. 7, 2000, pages 1553-1571, XP009024282 ISSN: 0967-8298

## Description

La présente invention a pour objet des dérivés de 5,6-diphénylpyridine-3-carboxamide, leur préparation et leur application en thérapeutique.

Des dérivés de 5,6-diphényl-2-pyridine sont décrits dans la demande de brevet internationale publiée sous le n° WO 92/02513. Ces composés sont présentés comme ayant une activité antithrombotique, vasodilatatrice, antiinflammatoire.

Des dérivés de 2,3-diphénylpyridine antagonistes ou agonistes inverses des récepteurs aux cannabinoides CB₁ sont décrits dans la demande de brevet internationales WO 2003/082191.

On a maintenant trouvé des nouveaux dérivés de 5,6-diphényl-3-pyridine carboxamide qui possèdent des propriétés antagonistes des récepteurs aux cannabinoïdes CB₁.

Ainsi la présente invention a pour objet des composés de formule : 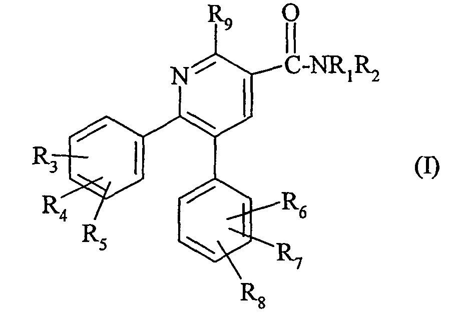 dans laquelle :
- R₁ représente l'hydrogène ou un (C₁-C₄)alkyle ;
- R₂ représente :
   - un radical hétérocyclique monoazoté, saturé, de 5 à 7 atomes, l'atome d'azote étant substitué par un groupe (C₁-C₄)alcanoyle ;
   - un groupe NR₁₀R₁₁ ;
   - un radical carbocyclique non aromatique en C₃-C₁₀ substitué plus de trois fois par un groupe (C₁-C₄)alkyle ;
   - un radical carbocyclique non aromatique en C₁₁-C₁₂, non substitué ou substitué une ou plusieurs fois par un groupe (C₁-C₄)alkyle ;
   - un radical hétérocyclique monooxygéné, saturé de 5 à 7 atomes, substitué plus de 3 fois par un groupe (C₁-C₄)alkyle ;
- ou R₁ et R₂ ensemble avec l'atome d'azote auquel ils sont liés constituent un radical pipéridin-1-yle disubstitué en 4- par un groupe phényle ou benzyle et par un groupe (C₁-C₄)alkyle, (C₁-C₄₎alcoxy, cyano, aminocarbonyle, (C₁-C₄)alkylaminocarbonyle, di(C₁-C₄)alkylaminocarbonyle, (C₁-C₃)alcanoyle ou (C₁-C₃)alcanoylamino ; les groupes phényles ou benzyles substituants le radical pipéridin-1-yle étant non substitués ou substitués par un atome d'halogène et/ou un groupe méthyle et/ou trifluorométhyle ;
- R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ;
- R₉ représente un atome d'hydrogène, un groupe (C₁-C₄)alkyle ou cyano ou un groupement CH₂OH, CH₂CN, ou CH₂O(C₁-C₄)alkyle ;
- R₁₀ et R₁₁ ensemble avec l'atome d'azote auquel ils sont liés constituent un radical hétérocyclique, saturé ou insaturé, de 7 à 10 atomes, contenant ou non un deuxième hétéroatome choisi parmi O ou N, ledit radical étant non substitué ou substitué une ou plusieurs fois par un groupe (C₁-C₄)alkyle, hydroxyle, (C₁-C₄)alcoxy, méthoxy(C₁-C₂)alkylène, ou substitué par un spirocyclobutane, un spirocyclopentane ou un spirocyclohexane ;
- ou R₁₀ et R₁₁ ensemble avec l'atome d'azote auquel ils sont liés constituent un radical hétérocyclique, saturé ou insaturé, de 5 à 6 atomes, contenant un deuxième atome d'azote, ledit radical étant substitué une ou plusieurs fois par un groupe méthoxy(C₁-C₂)alkylène, ou substitué par un spirocyclobutane, un spirocyclopentane ou un spirocyclohexane ;
- ou R₁₀ et R₁₁ ensemble avec l'atome d'azote auquel ils sont liés constituent un radical hétérocyclique, saturé ou insaturé, de 5 à 6 atomes, contenant ou non un atome d'oxygène, ledit radical étant substitué une ou plusieurs fois par un groupe (C₁-C₄)alkyle, hydroxyle, (C₁-C₄)alcoxy, méthoxy(C₁-C₂)alkylène, ou substitué par un spirocyclobutane, un spirocyclopentane ou un spirocyclohexane ;
ainsi que leurs sels, leurs solvats et leurs hydrates.

Les composés de formule (I) peuvent comporter un ou plusieurs atomes de carbone asymétriques. Ils peuvent donc exister sous forme d'énantiomères ou de diastéréoisoméres. Ces énantiomères, diastéréoisomères, ainsi que leurs mélanges, y compris les mélanges racémiques font partie de l'invention.

Les composés de formule (I) peuvent exister à l'état de bases ou de sels d'addition à des acides. Ces sels sont avantageusement préparés avec des sels pharmaceutiquement acceptables mais les sels d'autres acides utiles, par exemple, pour la purification ou l'isolement des composés de formule (I) font également partie de l'invention.

Parmi les composés de formule (I), on distingue les composés dans lesquels :
- R₁ représente l'hydrogène ou un (C₁-C₄)alkyle ;
- R₂ représente :
   - un radical hétérocyclique monoazoté, saturé, de 5 à 7 atomes, l'atome d'azote étant substitué par un groupe (C₁-C₄)alcanoyle ;
   - un groupe NR₁₀R₁₁ ;
   - un radical carbocyclique non aromatique en C₁₁-C₁₂, non substitué ou substitué une ou plusieurs fois par un groupe (C₁-C₄)alkyle ;
   - un radical hétérocyclique monooxygéné, saturé de 5 à 7 atomes, substitué plus de 3 fois par un groupe (C₁-C₄)alkyle ;
- ou R₁ et R₂ ensemble avec l'atome d'azote auquel ils sont liés constituent un radical pipéridin-1-yle disubstitué en 4- par un groupe phényle ou benzyle et par un groupe (C₁-C₄)alkyle ou (C₁-C₃)alcanoyle ; les groupes phényles ou benzyles substituants le radical pipéridin-1-yle étant non substitués ou substitués par un atome d'halogène et/ou un groupe méthyle ;
- R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ;
- R₉ représente un atome d'hydrogène, un groupe (C₁-C₄)alkyle ou cyano ou un groupement CH₂OH ou CH₂O(C₁-C₄)alkyle ;
- R₁₀ et R₁₁ ensemble avec l'atome d'azote auquel ils sont liés constituent un radical hétérocyclique, saturé ou insaturé, de 7 à 10 atomes, contenant ou non un deuxième hétéroatome choisi parmi O ou N, ledit radical étant non substitué ou substitué une ou plusieurs fois par un groupe (C₁-C₄)alkyle, hydroxyle, ou (C₁-C₄)alcoxy, méthoxy(C₁-C₂)alkylène, ou substitué par un spirocyclobutane, un spirocyclopentane ou un spirocyclohexane ;
- ou R₁₀ et R₁₁ ensemble avec l'atome d'azote auquel ils sont liés constituent un radical hétérocyclique, saturé ou insaturé, de 5 à 6 atomes, contenant un deuxième atome d'azote, ledit radical étant substitué une ou plusieurs fois par un groupe méthoxy(C₁-C₂)alkylène, ou substitué par un spirocyclobutane, un spirocyclopentane ou un spirocyclohexane ;
- ou R₁₀ et R₁₁ ensemble avec l'atome d'azote auquel ils sont liés constituent un radical hétérocyclique, saturé ou insaturé, de 5 à 6 atomes, contenant ou non un atome d'oxygène, ledit radical étant substitué une ou plusieurs fois par un groupe (C₁-C₄)alkyle, hydroxyle, (C₁-C₄)alcoxy, méthoxy(C₁-C₂)alkylène, ou substitué par un spirocyclobutane, un spirocyclopentane ou un spirocyclohexane ;
ainsi que leurs sels, leurs solvats et leurs hydrates.

Par groupe alkyle, on entend un radical linéaire ou ramifié, tel que en particulier : méthyle, éthyle, propyle, isopropyle, butyle, isobutyle, *tert*-butyle, n-péntyle, isopentyle, n-hexyle, isohexyle, le groupe méthyle étant préféré pour un (C₁-C₄)alkyle, les groupes *tert*-butyle, 2-méthylbutyl-2, 3,3-diméthylbutyl-2, étant préférés pour un (C₃-C₇)alkyle.

Par groupe alcoxy, on entend un radical linéaire ou ramifié, le groupe méthoxy étant préféré.

Par atome d'halogène, on entend un atome de fluor, de chlore, de brome ou d'iode ; les atomes de fluor, chlore ou brome étant préférés.

Les radicaux carbocycliques non aromatiques en C₃-C₁₂ comprennent les radicaux mono ou polycycliques, condensés ou pontés. Les radicaux monocycliques incluent les cycloalkyles par exemple cyclopropyle, cyclobutyle, cyclopentyle, cyclohexyle, cycloheptyle, cyclooctyle ; le cyclohexyle et le cyclopentyle étant préférés. Les radicaux di- ou tricycliques condensés, pontés ou spiraniques, incluent par exemple les radicaux norbomyle, bornyle, isobornyle, noradamantyle, adamantyle, spiro[5.5]undécanyle, bicyclo[2.2.1]heptanyle ; l'adamantyle étant préférés.

Par radical hétérocyclique, saturé ou insaturé, de 5 à 10 atomes, contenant ou non un deuxième hétéroatome tel que O ou N, on entend des radicaux tel que morpholin-4-yle, pipéridin-1-yle, pipérazin-1-yle, pyrrolidin-1-yle, 3,6-dihydropyridin-1-yle, octahydrocyclopenta[c]pyrrol-2-yle, les radicaux pipéridin-1-yle et morpholin-4-yle étant préférés.

Par radical hétérocyclique monoazoté, saturé, de 5 à 7 atomes, on entend un radical tel que pipéridin-4-yle ou pyrrolidin-3yle, le radical pipéridin-4-yle étant préféré.

Par radical hétérocyclique monooxygéné, saturé de 5 à 7 atomes, on entend un radical tel que tétrahydrofuranyle, tétrahydro-2*H*-pyranyle, oxepanyle : le tétrahydrofuranyle étant préféré.

Selon la présente invention, on préfère les composés de formule (I) dans laquelle :
- R₁ représente l'hydrogène ;
- R₂ représente : . un groupe NR₁₀R₁₁,
   - un radical carbocyclique non aromatique en C₁₁-C₁₂, non substitué ou substitué une ou plusieurs fois par un groupe méthyle ;
- ou R₁ et R₂ ensemble avec l'atome d'azote auquel ils sont liés constituent un radical pipéridin-1-yle disubstitué en 4- par un groupe phényle ou benzyle et par un groupe (C₁-C₄)alkyle ou (C₁-C₃)alcanoyle ;
- et/ou R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ;
- et/ou R₉ représente un atome d'hydrogène ou un groupe méthyle, méthoxyméthylène ou cyano ;
- R₁₀ et R₁₁ ensemble avec l'atome d'azote auquel ils sont liés constituent un radical hétérocyclique, saturé ou insaturé, de 5 à 10 atomes, contenant ou non un atome d'oxygène, ledit radical étant non substitué ou substitué une ou plusieurs fois par un groupe méthyle ;
ainsi que leurs sels, leurs solvats et leurs hydrates.

Parmi les composés objets de l'invention, on peut citer les composés préférés qui se définissent par les valeurs suivantes des substituants :
- R₁ représente un atome d'hydrogène ;
- et R₂ représente le groupe 3-amino-2,2,5,5-tétraméthyltétrahydrofurane ;
- ou R₁ et R₂ ensemble avec l'atome d'azote auquel ils sont liés représentent un groupe 4-acétyl-4-phénylpipéridin-1-yle ;
- et/ou au moins l'un des substituants R₃, R₄, R₅ représente un atome d'halogène, préférentiellement le chlore ou le brome ou un groupe méthoxy ;
- et/ou au moins l'un des substituants R₆, R₇, R₈ représente un atome d'halogène, préférentiellement le chlore ou le brome ;
- et/ou R₉ représente un groupe méthyle ou méthoxyméthyle.

Tout particulièrement, les composés suivants sont préférés : le 1-(1-(6-(4-chlorophényl)-5-(2,4-dichlorophényl)-2-méthylpyridin-3-yl)-4-phénylpipéridin-4-yl) éthanone, le 6-(4-chlorophényl)-5-(2,4-dichlorophényl)-2-(méthoxyméthyl-N-(2,2,5,5-tetraméthyltétrahydrofuran-3-yl)nicotinamide, le -5-(4-chlorophényl)-6-(2,4-dichlorophényl)-2-méthyl-N-(2,2,5,5-tétraméthyltétrahydofuran-3-yl)pyridine-3-carboxamide, le 1-(1-(5-(4-bromophényl)-6-(2,4-dichlorophényl)-2-(méthylpyridin-3-yl)carbonyl)-4-phénylpipéridin-4-yl)éthanone, le 6-(4-bromophényl)-5-(2,4-dichlorophényl)-2-méthyl-N-(2,2,5,5-tétraméthyltétrahydrofuran-3-yl)nicotinamide).

La présente invention a également pour objet un procédé de préparation des composés selon l'invention.

Ce procédé est caractérisé en ce que on traite un dérivé fonctionnel de l'acide 5,6-diphényl-2-pyridinecarboxylique de formule : 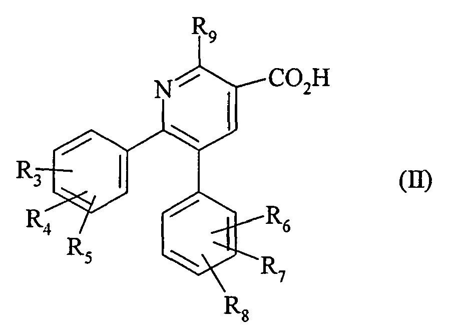 dans laquelle R₃, R₄, R₅, R₆, R₇, R₈ et R₉ sont tels que définis pour (I) avec une amine de formule HNR₁R₂ (III) dans laquelle R₁ et R₂ sont tels que définis pour (I). Eventuellement, on transforme le composé ainsi obtenu en un de ses sels ou solvats.

Comme dérivé fonctionnel de l'acide (II) on peut utiliser le chlorure d'acide, l'anhydride, un anhydride mixte, un ester alkylique en C₁-C₄ dans lequel l'alkyle est droit ou ramifié, un ester activé, par exemple l'ester de *p*-nitrophényle, ou l'acide libre opportunément activé, par exemple, avec le N,N-dicyclohexylcarbodiimide ou avec l'hexafluorophosphate de benzotriazol-1-yloxotris(diméthylamino)phosphonium (BOP) ou hexafluorophosphate de benzotriazol-1-yloxotris(pyrrolidino)phosphonium (PyBOP).

Ainsi dans le procédé selon l'invention, on peut faire réagir le chlorure de l'acide pyrazole-3-carboxylique, obtenu par réaction du chlorure de thionyle sur l'acide de formule (II), avec une amine HNR₁R₂, dans un solvant inerte, tel qu'un solvant chloré (le dichlorométhane, le dichloroéthane, le chloroforme par exemple), un éther (tétrahydrofurane, dioxane par exemple), ou un amide (N,N-diméthylformamide par exemple) sous une atmosphère inerte, à une température comprise entre 0°C et la température, en présence d'une amine tertiaire telle que la triéthylamine, la N-méthylmorpholine ou la pyridine.

Une variante consiste à préprarer l'anhydride mixte de l'acide de formule (II) par réaction du chloroformiate d'éthyle avec l'acide de formule (II), en présence d'une base telle que la triéthylamine, et à le faire réagir avec une amine HNR₁R₂, dans un solvant tel que le dichlorométhane, sous une atmosphère inerte, à la température ambiante, en présence d'une base telle que la triéthylamine.

Les composés de formule (II) peuvent être préparés par différents procédés selon la valeur du substituant R₉.

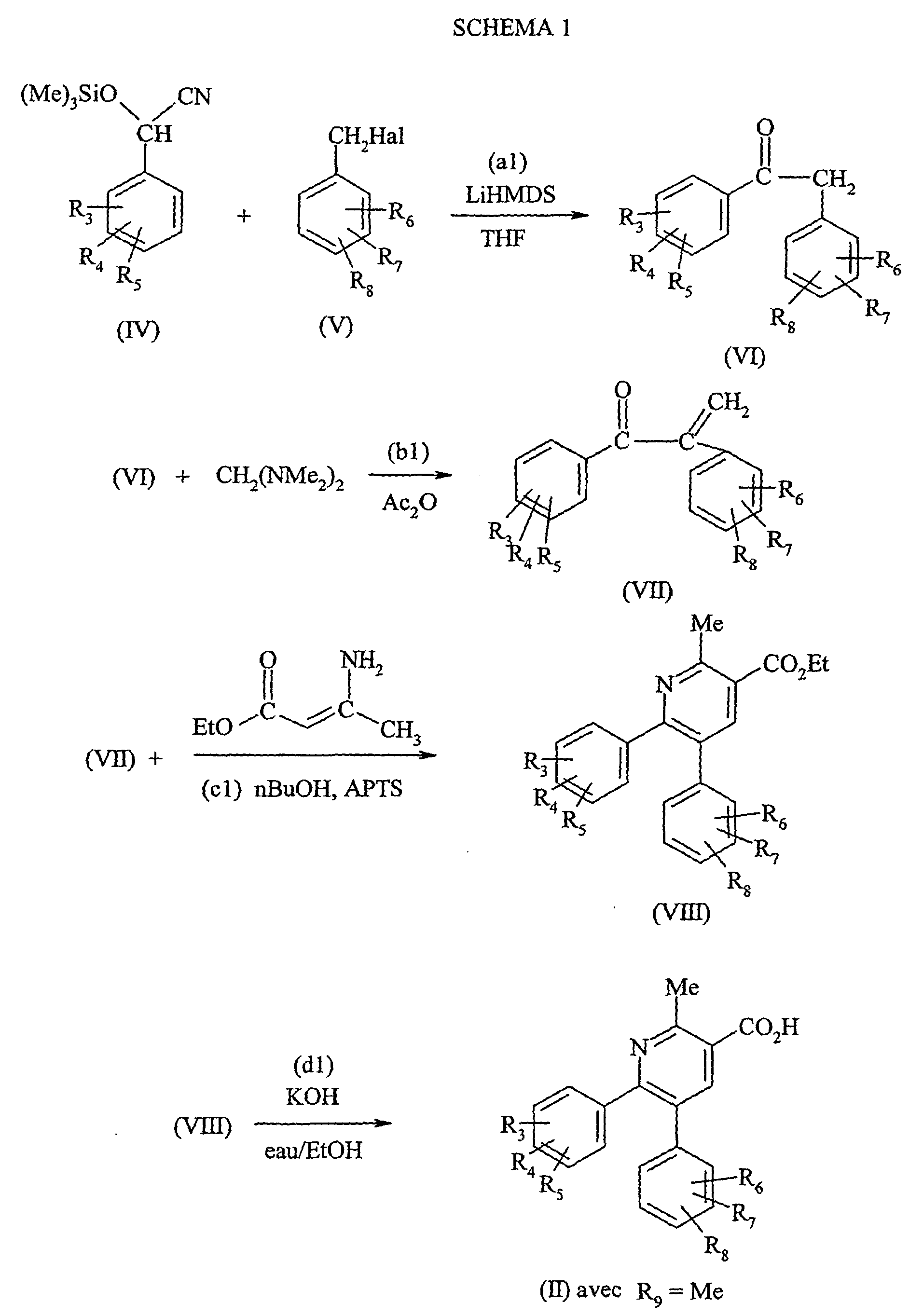

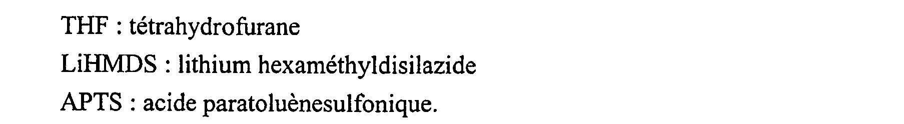

Le schéma 1 explicite un mode de préparation des composés de formule (II) dans laquelle R₉ = (C₁-C₄)alkyle, plus spécifiquement R₉ = Me.

L'étape a1) est effectuée dans un solvant anhydre tel que le THF, elle est initiée à basse température (-78°C) puis on laisse la température revenir à température ambiante (TA).

L'étape b1) est effectuée en présence d'anhydride acétique, à une température comprise entre TA et 100°C.

L'étape c1) est effectuée dans un solvant alcoolique tel que le n-butanol, en chauffant à reflux du solvant pendant 6 heures en présence d'un catalyseur tel que l'acide para-toluènesulfonique.

A l'étape d1), on réalise l'hydrolyse de l'ester par chauffage en milieu basique aqueux, dans un solvant tel que l'éthanol.

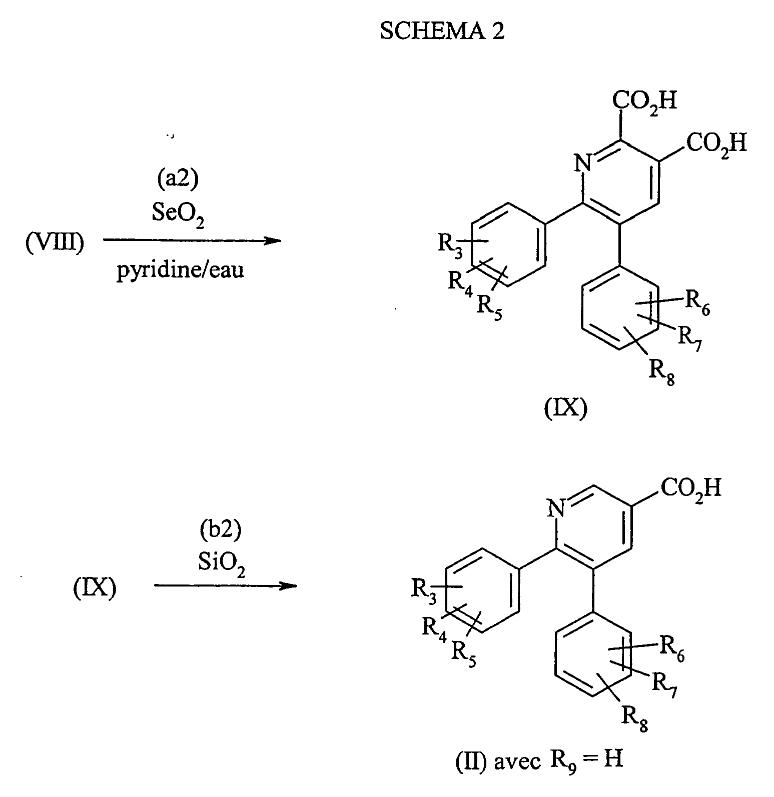

Pour préparer un composé de formule (II) dans laquelle R₉ est un atome d'hydrogène, on traite l'ester de formule (VIII) par un agent oxydant tel que l'oxyde de sélénium dans de la pyridine en milieu aqueux, puis on traite le diacide formé par de la silice à chaud.

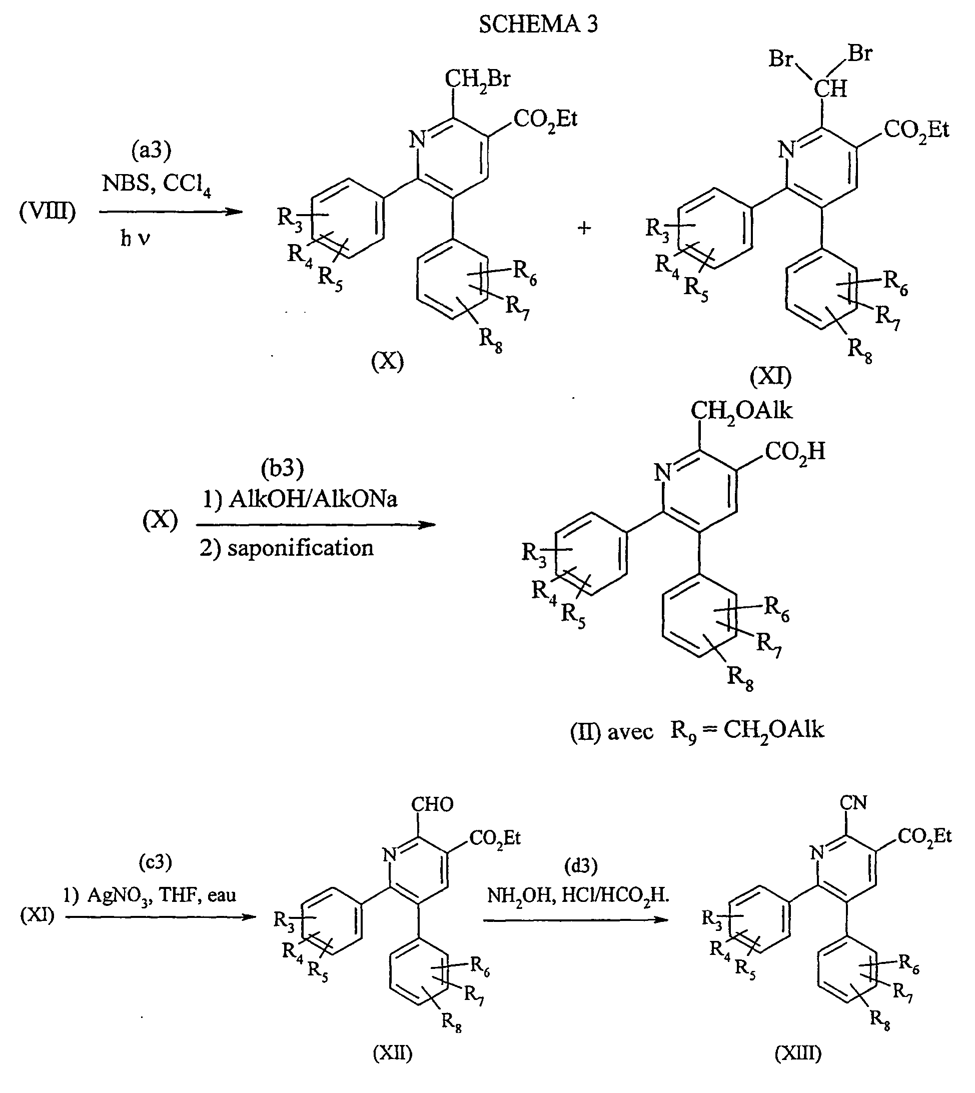

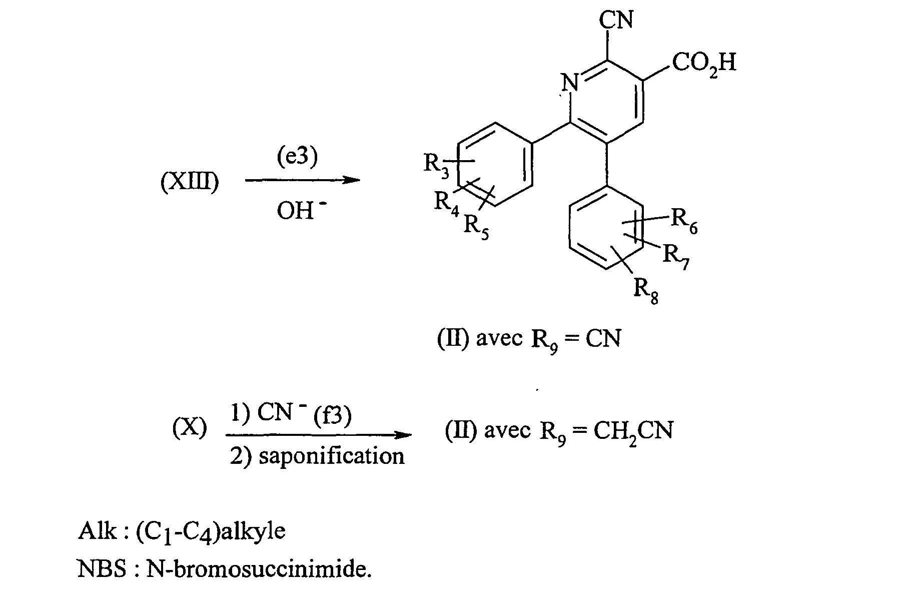

Les composés de formule (II) dans laquelle R₉ représente un groupe cyano, alcoxyméthylène ou cyanométhyle sont préparés à partir de l'ester de formule (VIII). Dans une première étape a3), le groupe R₉ = méthyle est substitué par 1 ou 2 atomes de brome par action du N-bromosuccinimide, à chaud, dans un solvant tel que CCl₄ en présence de rayonnement U.V. pour donner un dérivé monobromé (X) et un dérivé dibromé (XI).

A l'étape e3), le dérivé monobromé (X) est traité par un alcoolate de sodium dans un alcool, par exemple du méthylate de sodium dans le méthanol, puis saponifié en milieu basique, à chaud, pour donner l'acide de formule (II) dans laquelle R₉ est le groupe alcoxyméthylène.

A l'étape f3), le dérivé monobromé (X) est traité par le cyanure de tétraméthylammonium dans un solvant tel que le chloroforme, puis saponifié dans des conditions douces, par exemple par LiOH dans un mélange THF/eau pour donner l'acide de formule (II) dans laquelle R₉ est le groupe cyanométhyle.

Le dérivé dibromé (XI) est traité par du nitrate d'argent dans le THF aqueux pour former l'aldéhyde (XII) qui est ensuite traité par du chlorhydrate d'hydroxylamine dans l'acide formique pour donner un dérivé nitrile (XIII). L'ester (XIII) est traité en milieu basique, dans des conditions douces, de préférence par LiOH, pour donner l'acide de formule (II) dans laquelle R₉ est le groupe cyano.

Pour préparer un composé de formule (II) dans lequel R₉ = CH₂OH, le dérivé monobromé (X) est traité par de la soude, dans un mélange THF/eau, en chauffant à reflux, selon le schéma suivant.

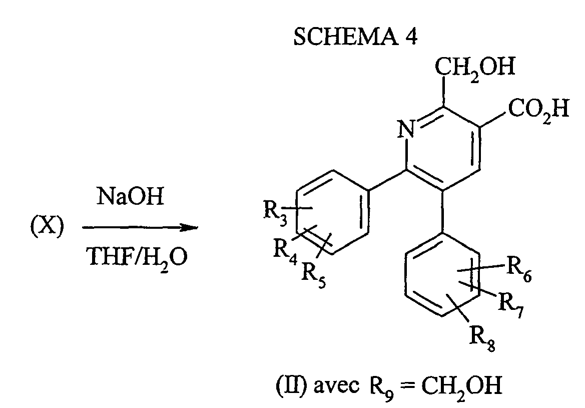

On peut également préparer un composé de formule (II) dans laquelle R₃ = CH₂OCH₃ par la méthode décrite dans le schéma 1 en utilisant à l'étape c1 un réactif approprié selon le schéma suivant :

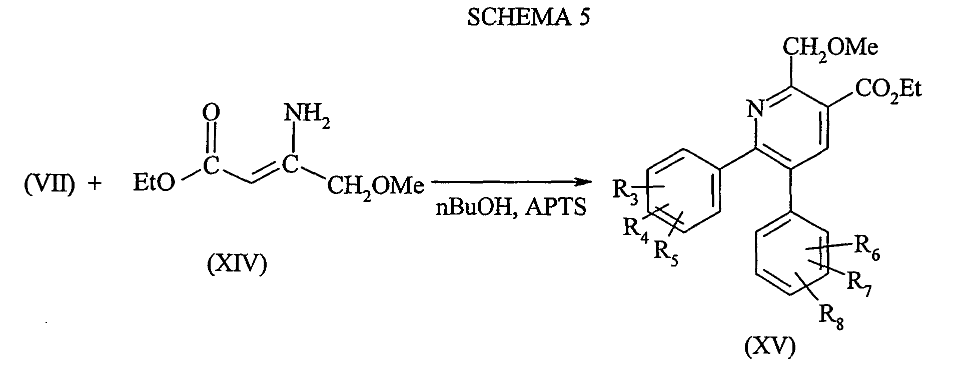

Le réactif (XIV) est obtenu delon le schéma réactionnel suivant :

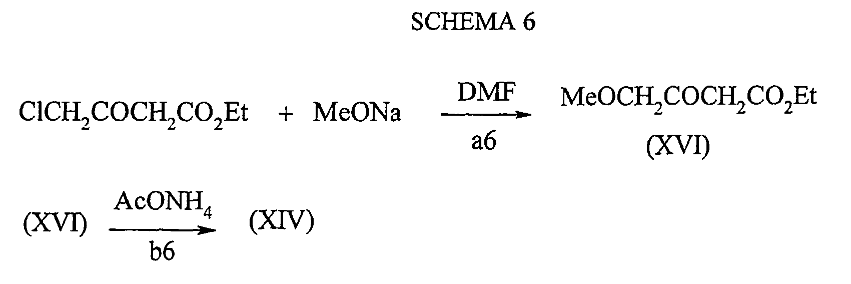

Les acides 5,6-diphényl-3-pyridinecarboxylique sont généralement connus ; l'acide 6-(4-chlorophényl)-5-(4-méthoxyphényl)-2-méthylpyridine-3-carboxylique et l'acide 5,6-bis-(4-méthoxyphényl)pyridine-3-carboxylique et leurs esters éthyliques sont décrits dans la demande de brevet internationale WO 2002/055502.

Les amines HNR₁R₂ sont connues ou préparées par des méthodes connues telles que celles décrites dans Chem. Ber., 1986, 119, 1413-1423.

Dans la présente description on utilise les abréviations suivantes :
DCM : dichlorométhane
LDA : lithium diisopropylamide
THF : tétrahydrofurane
TFA : acide trifluoroacétique
AIBN : azobis(isobutyronitrile)
TMSCl : triméthylchlorosilane
Et₂O ou Ether : éther éthylique
AcOEt : acétate d'éthyle
Ac₂O : anhydride acétique
nBuOH : n-butanol
APTS : acide para-toluènesulfonique
LiHMDS : lithium hexaméthyldisilazide
KHSO₄/K₂SO₄ : 0,5 mole KHSO₄/0,19 mole K₂SO₄ dans 1 litre d'eau
TEA : triéthylamine
BOP : hexafluorophosphate de benzotriazol-1-yloxotris(diméthylamino) phosphonium
PyBOP : hexafluorophosphate de benzotriazol-1-yloxotris(pyrrolidino) phosphonium
NBS : N-bromosuccinimide
TA : température ambiante
F : point de fusion.

Les composés selon l'invention sont analysés par couplage LC/UV/MS (chromatographie liquide/détection UV/spectrométrie de masse). On mesure le pic moléculaire (M⁺) et le temps de rétention (t) en minutes.

On utilise une colonne Symmetry C18, commercialisée par Waters, de 2,1 x 50 mm, 3,5 µm, à température ambiante, débit 0,4 mL/minute.

L'éluant est composé comme suit :
- solvant A : 0,005 % d'acide trifluoroacétique (TFA) dans l'eau
- solvant B : 0,005 % de TFA dans l'acétonitrile.

Gradient : Le pourcentage de solvant B varie de 0 à 90 % en 10 minutes avec un plateau à 90 % de B pendant 5 minutes.

La détection UV est effectuée à 210 nm ± 8 nm et la détection de masse en mode ionisation positif à pression atmosphérique.

Les spectres de résonance magnétique nucléaire (RMN) sont enregistrés à 200 MHz dans le DMSO-d₆. Pour l'analyse des spectres on utilise les abréviations suivantes : s : singulet ; d : doublet ; m : massif : se : singulet élargi ; dd : doublet de doublet ; mt : multiplet.

### Préparation 1

### Acide 5-(2,4-dichlorophényl)-6-(4-chlorophényl)-2-méthylpyridine-3-carboxylique.

### A) 1-(4-chlorophényl)-2-(2,4-dichlorophényl)éthanone.

Dans 245 ml de THF à 0°C on place 32 g de LiHMDS et on ajoute lentement à -78°C, 40 g de (4-chlorophényl)((triméthylsilyl)oxy)acétonitrile puis 32,64 g de 2,4-dichloro-1-(chlorométhyl)benzène. On laisse la température revenir à TA pendant une nuit, puis la réaction est hydrolysée par 170 ml d'une solution d'HCl 3N avec piégeage des gaz dans une solution de KOH (4N). Après décantation, la phase organique est évaporée puis reprise dans du DCM et agitée 1 heure avec 170 ml de NaOH (2N). On évapore le DCM puis le composé attendu cristallise dans le pentane. On obtient 38,6g,F=119°C.

### B) 1-(4-chlorophényl)-2-(2,4-dichlorophényl)prop-2-en-1-one.

On mélange à TA 10 g du composé de l'étape précédente, 17 ml de N,N,N,N-tétraméthylméthanediamine et 17 ml d'anhydride acétique ; on chauffe à 90°C pendant 3 heures puis on laisse revenir à TA. Le mélange est versé dans la glace pilée puis filtré. Le solide est séché sous vide. On obtient 10 g du composé attendu, F = 89°C.

### C) Ester éthylique de l'acide 5-(2,4-dichlorophényl)-6-(4-chlorophényl)-2-méthyl pyridine-3-carboxylique.

On prépare, dans 60 ml de n-butanol, un mélange contenant 7 g du composé de l'étape précédente, 2,62 g de 3-aminobut-2-ènoate d'éthyle et 140 mg d'acide *para-*toluènesulfonique puis on chauffe pendant 24 heures à reflux du solvant. On évapore le solvant au trois-quart puis on ajoute 80 ml de pentane à 0°C. On filtre le précipité formé et on concentre le filtrat. Le résidu est chromatographié sur silice en éluant par un mélange cyclohexane/AcOEt (90/10 ; v/v). On obtient 7 g du composé attendu, F = 114°C.

### D) Acide 5-(2,4-dichlorophényl)-6-(4-chlorophényl)-2-méthylpyridine-3-carboxylique.

On place 6 g de l'ester obtenu à l'étape précédente dans 300 ml d'EtOH et on ajoute 8 g de potasse. Après quelques minutes d'agitation, on ajoute 5 ml d'eau et on chauffe à reflux pendant 19 heures. On concentre le milieu réactionnel puis on reprend par Et₂O et de l'eau. La phase aqueuse est acidifiée à pH = 1 par HCl à 10 % puis on extrait au DCM et sèche sur Na₂SO₄. Après lavage à l'heptane, on obtient 5,4 g du composé attendu.

RMN : 2,83 ppm : s : 3H ; 7,2-7,6 ppm : m : 5H ; 7,68 ppm : s : 1H ; 8,11 ppm : s : 1H ; 13,2-13,7 ppm : se : 1H.

### Préparation 2

### Acide 6-(4-chlorophényl)-5-(2,4-dichlorophényl)pyridine-3-carboxylique.

### A) Acide 6-(4-chlorophényl)-5-(2,4-dichlorophényl)pyridine-2,3-dicarboxylique.

On place 2 g de l'acide obtenu à la Préparation 1 dans 7,2 ml de pyridine et 0,8 ml d'eau et on ajoute 1,44 g de dioxide de sélénium puis on chauffe à reflux pendant 3 jours. La solution obtenue est filtrée puis lavée à l'acide acétique. Après évaporation des solvants, on ajoute de la glace pilée et on maintient l'agitation pendant 2 heures. Le solide formé est filtré, lavé à l'éther puis séché à l'étuve. Le filtrat est concentré puis filtré sur silice en éluant au DCM. Le produit attendu est mis en réaction tel quel à l'étape suivante.

### B) Acide 6-(4-chlorophényl)-5-(2,4-dichlorophényl)pyridine-3-carboxylique.

Le produit obtenu à l'étape précédente est solubilisé dans le minimum de DCM et imprégné sur 25 g de silice. Après évaporation du solvant, la silice est chauffée à 120°C pendant 8 heures. On extrait le produit formé par DCM + EtOH puis EtOH pur. Après évaporation des solvants, on chromatographie sur silice en éluant par CHCl₃ puis CHCl₃/MeOH/AcOH (95/3/1,5 ; v/v/v). On obtient 544 mg du composé attendu.

RMN : 5,76 ppm : s : 1H ; 7,2-7,6 ppm : m : 5H ; 7,69 ppm : s : 1H ; 8,19 ppm : s : 1H ; 9,22 ppm : s : 1H ; 13,2-13,9 ppm : se : 1H.

### Préparation 3

### Acide 6-(4-chlorophényl)-5-(2,4-dichlorophényl)-2-méthoxyméthylpyridine-3-carboxylique.

### A) Ester éthylique de l'acide 2-bromométhyl-6-(4-chlorophényl)-5-(2,4-dichlorophényl)pyridine-3-carboxylique.

On place 3,8 g d'ester obtenu à la Préparation 1, étape C, dans 39 ml de CCl₄ avec 7,07 g de NBS et on ajoute 10 mg d'AIBN. On chauffe à reflux pendant 24 heures puis on maintient le chauffage sous irradiation U.V. pendant 8 heures. Après refroidissement, on filtre le milieu réactionnel puis le filtrat est chromatographié sur silice en éluant par le DCM ; on obtient 3,1 g d'ester éthylique de l'acide 2,2-dibromométhyl-6-(4-chlorophényl)-5-(2,4-dichlorophényl)pyridine-3-carboxylique et 1 g d'ester éthylique de l'acide 2-bromométhyl-6-(4-chlorophényl)-5-(2,4-dichlorophényl)pyridine-3-carboxylique.

### B) Ester éthylique de l'acide 6-(4-chlorophényl)-5-(2,4-dichlorophényl)-2-méthoxyméthylpyridine-3-carboxylique.

On place 1 g du composé monobromé obtenu à l'étape précédente dans 30 ml de MeOH puis on ajoute 0,13 g de méthanolate de sodium et on chauffe à reflux pendant 24 heures. Le milieu réactionnel est hydrolysé par une solution d'HCl à 10 % puis on évapore le solvant et reprend par AcOEt puis lave à l'eau. Après séchage sur MgSO₄, on concentre puis on chromatographie sur silice en éluant par le mélange cyclohexane/AcOEt (90/10 ; v/v). On obtient 840 mg de l'ester attendu.

### C) Acide 6-(4-chlorophényl)-5-(2,4-dichlorophényl)-2-méthoxyméthylpyridine-3-carboxylique.

On place 800 mg de l'ester obtenu à l'étape précédente dans 37 ml d'EtOH et l'on ajoute 1,02 g de KOH puis on laisse 1 heure sous agitation à TA et on ajoute 600 µl d'eau. On chauffe 6 heures à reflux puis on évapore l'éthanol. On reprend par un mélange Et₂O/eau puis on décante. La phase aqueuse est acidifiée à pH = 2 par addition d'HCl 1N. On obtient 890 mg de l'acide attendu.

RMN : 3,36 ppm : s : 3H ; 4,86 ppm : s : 2H ; 7,2-7,4 ppm : m : 4H ; 7,48 ppm : s : 1H ; 7,68 : s : 1H ; 8,08 ppm : s : 1H ; 13,0-13,8 : se : 1H.

### Préparation 3bis

### Acide 6-(4-chlorophényl)-5-(2,4-dichlorophényl)-2-méthoxyméthylpyridine-3-carboxylique.

Ce composé peut être également préparé selon le mode opératoire décrit ci-après.

### A) 4-Méthoxy-3-oxobutanoate d'éthyle.

Dans 200ml de DMF, on introduit sous azote 14 g d'hydrure de sodium, on refroidit à 0°C et on ajoute goutte à goutte 6 ml de méthanol dans 100 ml de DMF et on laisse sous agitation 1 heure à 0°C puis une heure à TA. A 0°C, on ajoute 17,32 g de 4-chloro-3-oxobutanoate dans 100ml de DMF et on laisse une nuit sous agitation. On verse le milieu réactionnel dans de l'eau à 0°C puis on extrait au DCM, lave 3 fois à l'eau puis 3 fois par une solution saturée de NaCl. La phase aqueuse est reprise, acidifiée à pH > 4 puis extraite par le DCM, lavée à l'eau 3 fois, puis par une solution saturée de NaCl. On sèche puis concentre à sec. On chromatographie sur Cetite^{®} en éluant par cyclohexane/AcOEt (80/20 ; v/v). On obtient 3,90 g du composé attendu sous forme liquide.

### B) 3-Amino-4-méthoxybut-2-énoate d'éthyle.

On place sous azote le tamis moléculaire (3Å), 3,9 g du composé obtenu à l'étape précédente et 4 g d'acétate d'ammonium fraichement sublimé, dans 50 ml de cyclohexane, on chauffe à 80°C pendant 1 heure 30 minutes puis une nuit à TA. On filtre et on rince le filtrat par CHCl₃. On obtient 2,4 g du composé attendu.

### C) Ester éthylique de l'acide 6-(4-chlorophényl)-5-(2,4-dichlorophényl)-2-méthoxyméthylpyridine-3-carboxylique.

On dissout par chauffage 4,7 g du composé de la Préparation 1, étape B dans 25 ml de n-butanol ; on ajoute 2,29 g du composé obtenu à l'étape précédente dans 5 ml de n-butanol puis 0,25 g d'APTS et on chauffe à reflux pendant 19 heures. Le milieu réactionnel est trituré dans Et₂O à froid. On filtre l'insoluble et on concentre la phase organique puis on chromatographie sur silice en éluant par cyclohéxane/AcOEt (95/5 ; v/v). On obtient 3,4 g du composé attendu.

### G) Acide 6-(4-chlorophényl)-5-(2,4-dichlorophényl)-2-méthoxyméthylpyridine-3-carboxylique.

Ce composé est obtenu comme décrit à la Préparation 3, étape C) ci-dessus.

En suivant les modes opératoires décrits dans les préparations ci-dessus, on a également préparé les composés intermédiaires de formule (II) du tableau ci-après.

**TABLEAU 1**

| | | |
|---|---|---|
| 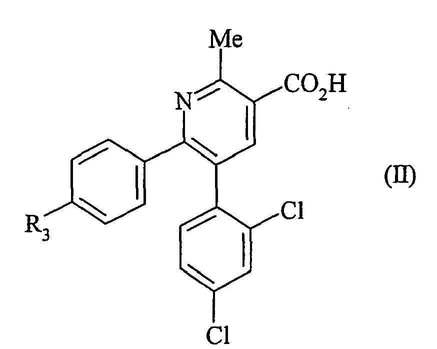 | | |

| Préparations | R₃ | F°C |
|---|---|---|
| 4 | OMe | 214°C |
| 5 | Br | 214,5°C |

### EXEMPLE 1 : Composé 3

### 1-(1-(6-(4-chlorophényl)-5-(2,4-dichlorophényl)-2-méthylpyridin-3-yl)-4-phénylpipéridin-4-yl)éthanone.

62,7 mg de l'acide obtenu à la Préparation 1 et 38,6 mg de 1-phényl-1-pipéridin-4-ylacetone sont mis en solution dans 2 ml de DCM et 67,5 ml de triéthylamine. Ensuite on ajoute 104mg de PyBOP, la réaction est laissée sous agitation à TA pendant 2 heures. Après évaporation des solvants, le brut est chromatographié sur silice selon le gradient d'éluant suivant : dichlorométhane pur puis dichlorométhane/méthanol (99/1 ; v/v), on obtient 52 mg du composé attendu.

### EXEMPLE 2 : Composé 10

### 6-(4-chlorophényl)-5-(2,4-dichlorophényl)-2-(méthoxyméthyl-N-(2,2,5,5-tetraméthyltétrahydrofuran-3-yl)nicotinamide.

On place 0,42 g de l'acide obtenu à la Préparation 3 dans 2 ml de DCM et on ajoute 0,34 g de 2,2,5,5-tetraméthyltetrahydrofuran-3-yl) amine, 0,30 g de TEA et 0,60 g de PyBOP et on laisse une nuit sous agitation sous azote. On ajoute une solution de KHSO₄/K₂SO₄, on agite vigoureusement puis on extrait au DCM. On lave la phase organique par une solution saturée de NaHCO₃ puis de l'eau. On sèche et concentre puis le résidu est chromatographié sur silice en éluant par cyclhéxane/AcoEt (3/1 ; v/v). On obtient 0,54 g du composé attendu.F = 67-69°C.

Le tableau qui suit illustre les structures chimiques et les propriétés physiques de quelques exemples de composé selon l'invention.

Dans ce tableau Me, Et, nPr, tBu, nPn représentent respectivement les groupes méthyle, éthyle, n-propyle, *tert*-butyle et n-pentyle.

**TABLEAU 2**

| | | | | | |
|---|---|---|---|---|---|
| 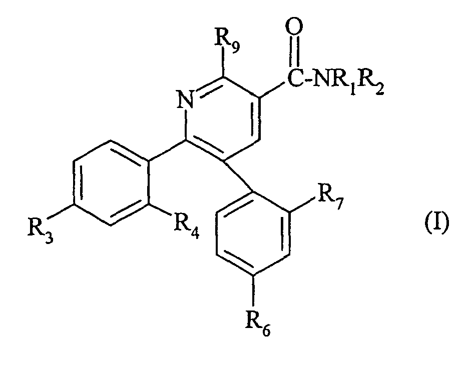 | | | | | |

| Composés | R₃, R₄ | R₆, R₇ | R₉ | NR₁R₂ | Caractérisation |
|---|---|---|---|---|---|
| 1 | 4-Cl | 2,4-diCl | Me | 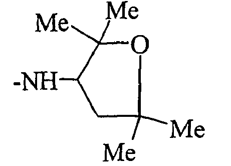 | M⁺ = 517 |
| | | | | | t = 9,88 |
| 2 | 4-Cl | 2,4-diCl | Me | 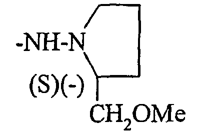 | M⁺ = 504 |
| | | | | | t = 8,94 |
| 3 | 4-Cl | 2,4-diCl | Me | 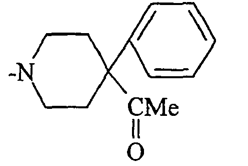 | M⁺ = 577 |
| | | | | | t = 10,57 |
| 4 | 4-Cl | 2,4-diCl | Me | 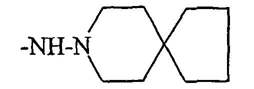 | M⁺ = 528 |
| | | | | | t = 10,63 |
| 5 | 4-Cl | 2,4-diCl | Me | 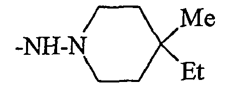 | M⁺ = 516 |
| | | | | | t = 10,47 |
| 6 | 4-OMe | 2,4-diCl | Me | 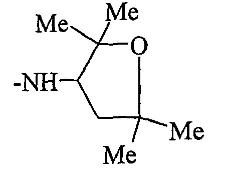 | F = 115,5°C |
| 7 | 4-Br | 2,4-diCl | Me | 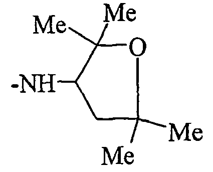 | F = 109,4°C |
| 8 | 2,4-diCl | 4-Br | Me | 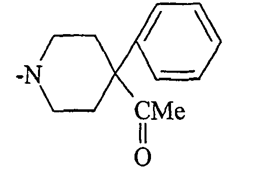 | F = 210°C |
| 9 | 4-Cl | 2,4-diCl | CH₂OMe | 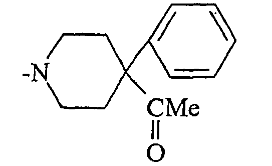 | F = 102°C |
| 10 | 4-Cl | 2,4-diCl | CH₂OMe | 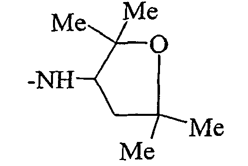 | F = 67°C |
| 11 | 4-Br | 2,4-diCl | Me | 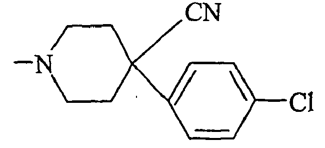 | F = 137°C |
| 12 | 4-Br | 2,4-diCl | Me | 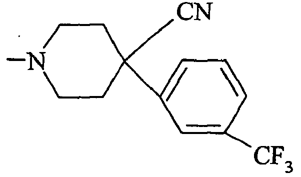 | F = 128°C |
| 13 | 4-Br | 2,4-diCl | Me | 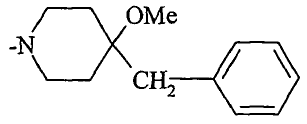 | F = 110°C |
| 14 | 4-Br | 2,4-diCl | Me | 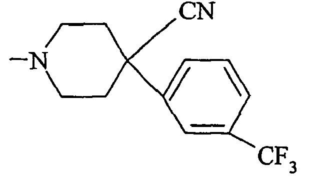 | F = 240°C |
| 15 | 4-Br | 2,4-diCl | Me | 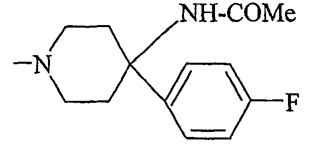 | F = 166°C |
| 16 | 4-Cl | 2,4-diCl | Me | 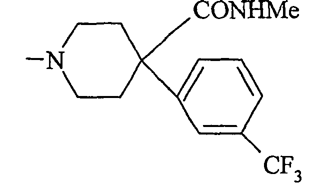 | F = 138°C |
| 17 | 4-Cl | 2,4-diCl | Me | 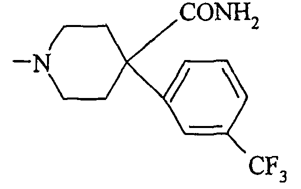 | F = 142°C |
| 18 | 4-Cl | 2,4-diCl | Me | 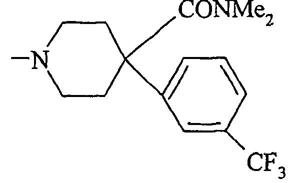 | F = 134°C |
| 19 | 4-Br | 2,4-diCl | Me | 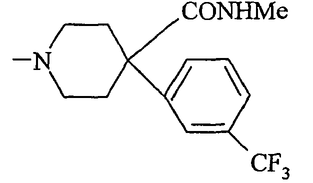 | M⁺ = 703 |
| | | | | | t = 10,57 |
| 20 | 4-Br | 2,4-diCl | Me | 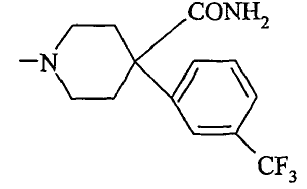 | M⁺ = 689 |
| | | | | | t = 11,05 |
| 21 | 4-Br | 2,4-diCl | Me | 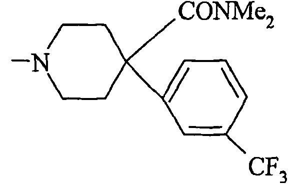 | M⁺= 717 |
| | | | | | t = 11,96 |

Les composés selon l'invention ont fait l'objet d'essais pharmacologiques permettant de déterminer leur effet antagoniste des récepteurs aux cannabinoïdes CB₁.

Les composés de formule (I) possèdent une très bonne affinité *in vitro* (IC₅₀ comprise en 5 nM et 1000 nM) pour les récepteurs aux cannabinoïdes CB₁, dans les conditions expérimentales décrites par M. Rinaldi-Carmona et al. (FEBS Letters, 1994, 350, 240-244).

La nature antagoniste des composés de formule (I) a été démontrée par les résultats obtenus dans les modèles de l'inhibition de l'adénylate-cyclase comme décrits dans M. Rinaldi-Carmona et al., J. Pharmacol. Exp. Therap., 1996, 278, 871-878 et M. Bouaboula et al., J. Biol. Chem., 1997, 272, 22330-22339.

Les composés selon l'invention ont été testés *in vivo* (binding ex vivo) chez la souris après l'administration intraveineuse et/ou orale d'un composé de l'invention, selon les conditions expérimentales décrites par Rinaldi-Carmona et al. (J. Pharmacol. Exp. Therap., 1998, 284, 644-650).

La toxicité des composés de formule (I) est compatible avec leur utilisation en tant que médicament.

Selon un autre de ses aspects, la présente invention concerne l'utilisation d'un composé de formule (I), ou de l'un de ses sels, solvats ou hydrates pharmaceutiquement acceptable, pour la préparation de médicaments destinés à traiter ou à prévenir les maladies impliquant les récepteurs aux cannabinoïdes CB₁.

Par exemple et de manière non limitative, les composés de formule (I) sont utiles comme médicaments psychotropes, notamment pour le traitement des désordres psychiatriques incluant l'anxiété, la dépression, les troubles de l'humeur, l'insomnie, les troubles délirants, les troubles obsessionnels, les psychoses en général, la schizophrénie, les troubles de l'attention et l'hyperactivité (TDAH), notamment chez les enfants hyperkinétiques (MBD), ainsi que pour le traitement des troubles liés à l'utilisation de substances psychotropes, notamment dans le cas d'un abus d'une substance et/ou de dépendance à une substance, y compris la dépendance alcoolique et la dépendance nicotinique.

Les composés de formule (I) selon l'invention peuvent être utilisés comme médicaments pour le traitement de la migraine, du stress, des maladies d'origine psychosomatique, des crises d'attaques de panique, de l'épilepsie, des troubles du mouvement, en particulier des dyskinésies ou de la maladie de Parkinson, des tremblements et de la dystonie.

Les composés de formule (I) selon l'invention peuvent également être utilisés comme médicaments dans le traitement des troubles mnésiques, des troubles cognitifs, en particulier dans le traitement des démences séniles, de la maladie d'Alzheimer, ainsi que dans le traitement des troubles de l'attention ou de la vigilance. De plus, les composés de formule (I) peuvent être utiles comme neuroprotecteurs, dans le traitement de l'ischémie, des traumatismes crâniens et le traitement des maladies neurodégénératives : incluant la chorée, la chorée de Huntington, le syndrome de Tourrette.

Les composés de formule (I) selon l'invention peuvent être utilisés comme médicaments dans le traitement de la douleur : les douleurs neuropathiques, les douleurs aiguës périphériques, les douleurs chroniques d'origine inflammatoire.

Les composés de formule (I) selon l'invention peuvent être utilisés comme médicaments dans le traitement des troubles de l'appétit, de l'appétence (pour les sucres, carbohydrates, drogues, alcools ou toute substance appétissante) et/ou des conduites alimentaires, notamment en tant qu'anorexigènes ou pour le traitement de l'obésité ou de la boulimie ainsi que pour le traitement du diabète de type II ou diabète non insulinodépendant et pour le traitement des dyslipidémies, du syndrome métabolique. De plus, les composés de formule (I) selon l'invention peuvent être utilisés en tant que médicaments dans le traitement des troubles gastro-intestinaux, des troubles diarrhéiques, des ulcères, des vomissements, des troubles vésicaux et urinaires, des troubles d'origine endocrinienne, des troubles cardio-vasculaires, de l'hypotension, du choc hémorragique, du choc septique, de la cirrhose chronique du foie, de l'asthme, de la bronchite chronique et de la broncho-pneumopathie chronique obstructive, du syndrome de Raynaud, du glaucome, des troubles de la fertilité, des phénomènes inflammatoires, des maladies du système immunitaire, en particulier autoimmunes et neuroinflammatoires tel que l'arthrite rhumatoïde, l'arthrite réactionnelle, les maladies entraînant une démyélinisation, la sclérose en plaque, des maladies infectieuses et virales telles que les encéphalites, des accidents vasculaires cérébraux ainsi qu'en tant que médicaments pour la chimiothérapie anticancéreuse et pour le traitement du syndrome de Guillain-Barré.

Selon la présente invention, les composés de formule (I) sont tout particulièrement utiles pour le traitement des troubles psychotiques, en particulier la schizophrénie, les troubles de l'attention et l'hyperactivité (TDAH), notamment chez les enfants hyperkinétiques (MBD) ; pour le traitement des troubles de l'appétit et de l'obésité pour le traitement des troubles mnésiques et cognitifs ; pour le traitement de la dépendance alcoolique, de la dépendance nicotinique, c'est à dire pour le sevrage alcoolique et pour le sevrage tabagique ; pour le traitement des dyslipidémies, du syndrome métabolique.

Selon un de ses aspects, la présente invention est relative à l'utilisation d'un composé de formule (I), de ses sels pharmaceutiquement acceptables et de leurs solvats ou hydrates pour le traitement des troubles et maladies indiqués ci-dessus.

Les composés de formule (I) selon l'invention peuvent être utilisés en association avec un ou plusieurs autres principes actifs utiles pour la prévention et/ou le traitement des maladies indiquées ci-dessus : à titre d'exemple de principes actifs pouvant être associés à un composé de formule (I), on peut citer les antipsychotiques, les anxiolytiques, des agents améliorant la mémoire, des agents anti-parkinson, des anti-épileptiques, des anorexigènes ou d'autres agents anti-obésité, des agonistes nicotiniques, des inhibiteurs de la monoamine oxidase, des analgésiques, des anti-inflammatoires, des antihypertenseurs tels que : antagonistes des récepteurs AT₁ de l'angiotensine II, inhibiteurs de l'enzyme de conversion, antagonistes calciques, beta-bloquants, des anti-diabétiques, des anti-hyperlipémiants, des anti-hypercholestérolémiants, des agonistes PPAR (de l'anglais peroxisome proliferator activated receptor).

Le composé selon l'invention est généralement administré en unité de dosage.

Lesdites unités de dosage sont de préférence formulées dans des compositions pharmaceutiques dans lesquelles le principe actif est mélangé avec un excipient pharmaceutique.

Ainsi, selon un autre de ses aspects, la présente invention concerne des compositions pharmaceutiques renfermant, en tant que principe actif, un composé de formule (I), un de ses sels pharmaceutiquement acceptables ou un de leurs solvats.

Le composé de formule (I) ci-dessus et ses sels ou solvats pharmaceutiquement acceptables peuvent être utilisés à des doses journalières de 0,01 à 100 mg par kg de poids corporel du mammifère à traiter, de préférence à des doses journalières de 0,02 à 50 mg/kg. Chez l'être humain, la dose peut varier de préférence de 0,05 à 4000 mg par jour, plus particulièrement de 0,1 à 1000 mg par jour selon l'âge du sujet à traiter ou le type de traitement, à savoir prophylactique ou curatif. Bien que ces dosages soient des exemples de situations moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Dans les compositions pharmaceutiques de la présente invention pour l'administration orale, sublinguale, inhalée, sous-cutanée, intramusculaire, intraveineuse, transdermique, locale ou rectale, le principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les formes d'administration topique, les implants, les formes d'administration sous-cutanée, intramusculaire, intraveineuse, intranasale ou intra-oculaire et les formes d'administration rectale.

Dans les compositions pharmaceutiques de la présente invention, le principe actif est généralement formulé en unités de dosage contenant de 0,05 à 1000 mg, avantageusement de 0,1 à 500 mg, de préférence de 1 à 200 mg dudit principe actif par unité de dosage pour les administrations quotidiennes.

A titre d'exemple, une forme unitaire d'administration d'un composé selon l'invention sous forme de comprimé peut comprendre les composants suivants :

| | | |
|---|---|---|
| Composé selon l'invention | : | 50,0 mg |
| Mannitol | : | 223,75 mg |
| Croscarmellose sodique | : | 6,0 mg |
| Amidon de maïs | : | 15,0 mg |
| Hydroxypropyl-méthylcellulose | : | 2,25 mg |
| Stéarate de magnésium | : | 3,0 mg |

Par voie orale, la dose de principe actif administrée par jour peut atteindre 0,01 à 100 mg/kg, en une ou plusieurs prises, préférentiellement 0,02 à 50 mg/kg.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un autre de ses aspects, concerne également une méthode de traitement des pathologies ci-dessus indiquées qui comprend l'administration, à un patient, d'une dose efficace d'un composé selon l'invention, ou un de ses sels pharmaceutiquement acceptables ou hydrates ou solvats.

## Revendications

1. Composé de formule : 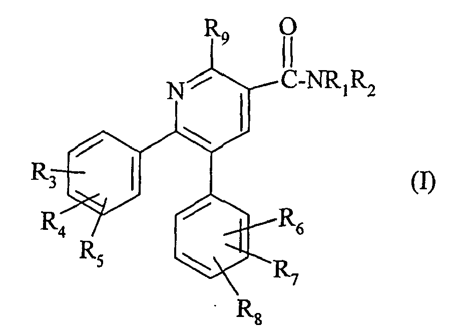 dans laquelle :
- R₁ représente l'hydrogène ou un (C₁-C₄)alkyle ;
- R₂ représente :
. un radical hétérocyclique monoazoté, saturé, de 5 à 7 atomes, l'atome d'azote étant substitué par un groupe alcanoyle ;
. un groupe NR₁₀-R₁₁ ;
. un radical carbocyclique non aromatique en C₃-C₁₀, substitué plus de 3 fois par un groupe (C₁-C₄)alkyle ;
. un radical carbocyclique non aromatique en C₁₁-C₁₂, non substitué ou substitué une ou plusieurs fois par un groupe (C₁-C₄)alkyle ;
. un radical hétérocyclique monooxygéné, saturé de 5 à 7 atomes, substitué plus de 3 fois par un groupe (C₁-C₄)alkyle ;
- ou R₁ et R₂ ensemble avec l'atome d'azote auquel ils sont liés constituent un radical pipéridin-1-yle disubstitué en 4- par un groupe phényle ou benzyle et par un groupe (C₁-C₄)alkyle, (C₁-C₄)alcoxy, cyano, aminocarbonyle, (C₁-C₄)alkylaminocarbonyle, di(C₁-C₄)alkylaminocarbonyle, (C₁-C₃)alcanoyle ou (C₁-C₃)alcanoylamino ; les groupes phényles ou benzyles substituants le radical pipéridin-1-yle étant non substitués ou substitués par un atome d'halogène et/ou un groupe méthyle et/ou trifluorométhyle ;
- R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ;
- R₉ représente un atome d'hydrogène, un groupe (C₁-C₄)alkyle ou cyano ou un groupement CH₂OH, CH₂CN ou CH₂O(C₁-C₄)alkyle ;
- R₁₀ et R₁₁ ensemble avec l'atome d'azote auquel ils sont liés constituent un radical hétérocyclique, saturé ou insaturé, de 7 à 10 atomes, contenant ou non un deuxième hétéroatome choisi parmi O ou N, ledit radical étant non substitué ou substitué une ou plusieurs fois par un groupe (C₁-C₄)alkyle, hydroxyle, (C₁-C₄)alcoxy, méthoxy(C₁-C₂)alkylène, ou substitué par un spirocyclobutane, un spirocyclopentane ou un spirocyclohexane ;
- ou R₁₀ et R₁₁ ensemble avec l'atome d'azote auquel ils sont liés constituent un radical hétérocyclique, saturé ou insaturé, de 5 à 6 atomes, contenant un deuxième atome d'azote, ledit radical étant substitué une ou plusieurs fois par un groupe méthoxy(C₁-C₂)alkylène, ou substitué par un spirocyclobutane, un spirocyclopentane ou un spirocyclohexane ;
- ou R₁₀ et R₁₁ ensemble avec l'atome d'azote auquel ils sont liés constituent un radical hétérocyclique, saturé ou insaturé, de 5 à 6 atomes, contenant ou non un atome d'oxygène, ledit radical étant substitué une ou plusieurs fois par un groupe (C₁-C₄)alkyle, hydroxyle, (C₁-C₄)alcoxy, méthoxy(C₁-C₂)alkylène, ou substitué par un spirocyclobutane, un spirocyclopentane ou un spirocyclohexane ;
à l'état de base ou de sel d'addition à un acide, ainsi qu'à l'état d'hydrate ou de solvat.

2. Composé de formule (I) selon la revendication 1 **caractérisé en ce que** :
- R₁ représente l'hydrogène ;
- R₂ représente : . un groupe NR₁₀R₁₁,
. un radical carbocyclique non aromatique en C₁₁-C₁₂, non substitué ou substitué une ou plusieurs fois par un groupe méthyle ;
- ou R₁ et R₂ ensemble avec l'atome d'azote auquel ils sont liés constituent un radical pipéridin-1-yle disubstitué en 4- par un groupe phényle ou benzyle et par un groupe (C₁-C₄)alkyle ou (C₁-C₃)alcanoyle ;
- et/ou R₃, R₄, R₅, R₆, R₇, R₈ représentent chacun indépendamment l'un de l'autre un atome d'hydrogène ou d'halogène, un groupe (C₁-C₆)alkyle, (C₁-C₆)alcoxy ou trifluorométhyle ;
- et/ou R₉ représente un atome d'hydrogène ou un groupe méthyle, méthoxyméthylène ou cyano ;
- R₁₀ et R₁₁ ensemble avec l'atome d'azote auquel ils sont liés constituent un radical hétérocyclique, saturé ou insaturé, de 5 à 10 atomes, contenant ou non un atome d'oxygène, ledit radical étant non substitué ou substitué une ou plusieurs fois par un groupe méthyle ;
à l'état de base ou de sel d'addition à un acide, ainsi qu'à l'état d'hydrate ou de solvat.

3. Composés de formule (I) selon la revendication 1 **caractérisé en ce que** :
- R₁ représente un atome d'hydrogène ;
- et R₂ représente le groupe 3-amino-2,2,5,5-tétraméthyltétrahydrofurane ;
- ou R₁ et R₂ ensemble avec l'atome d'azote auquel ils sont liés représentent un groupe 4-acétyl-4-phénylpipéridin-1-yle ;
- et/ou au moins l'un des substituants R₃, R₄, R₅ représentent un atome de chlore ou de brome ou un groupe méthoxy ;
- et/ou au moins l'un des substituants R₆, R₇, R₈ représente un atome de chlore ou de brome ;
- et/ou R₉ représente un groupe méthyle ou méthoxyméthyle,
à l'état de base ou de sel d'addition à un acide, ainsi qu'à l'état d'hydrate ou de solvat.

4. Composé de formule (I) selon la revendication 1 choisi parmi :
- le 1-(1-(6-(4-chlorophényl)-5-(2,4-dichlorophényl)-2-méthylpyridin-3-yl)-4-phénylpipéridin-4-yl) éthanone, le 6-(4-chlorophényl)-5-(2,4-dichlorophényl)-2-(méthoxyméthyl-N-(2,2,5,5-tetraméthyltétrahydrofuran-3-yl)nicotinamide,
- le -5-(4-chlorophényl)-6-(2,4-dichlorophényl)-2-méthyl-N-(2,2,5,5-tétraméthyl tétrahydofuran-3-yl)pyridine-3-carboxamide,
- le 1-(1-(5-(4-bromophényl)-6-(2,4-dichlorophényl)-2-(méthylpyridin-3-yl) carbonyl)-4-phénylpipéridin-4-yl)éthanone,
- le 6-(4-bromophényl)-5-(2,4-dichlorophényl)-2-méthyl-N-(2,2,5,5-tétraméthyl tétrahydrofuran-3-yl)nicotinamide),
à l'état de base ou de sel d'addition à un acide, ainsi qu'à l'état d'hydrate ou de solvat.

5. Procédé de préparation d'un composé de formule (I) selon l'une quelconque des revendications 1 à 4, **caractérisé en ce que** l'on traite un dérivé fonctionnel de l'acide 5,6-diphényl-3-pyridinecarboxylique de formule : 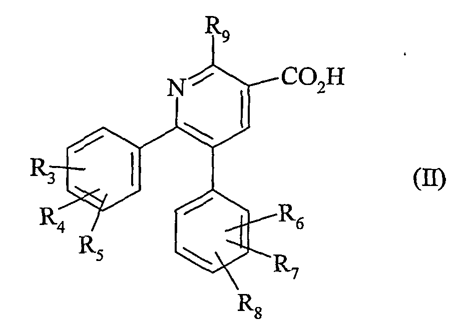 dans laquelle R₃, R₄, R₅, R₆, R₇, R₈ et R₉ sont tels que définis pour (I) à la revendication 1, avec une amine de formule HNR₁R₂ (III) dans laquelle R₁ et R₂ sont tels que définis pour (I) à la revendication 1.

6. Médicament **caractérisé en ce qu'**il comprend un composé de formule (I) selon l'une quelconque des revendications 1 à 4, ou un sel d'addition de ce composé à un acide pharmaceutiquement acceptable, ou encore un hydrate ou un solvat.

7. Composition pharmaceutique **caractérisé en ce qu'**elle comprend un composé de formule (I) selon l'une quelconque des revendications 1 à 4 ou un sel pharmaceutiquement acceptable, un hydrate ou un solvat de ce composé ainsi qu'au moins un excipient pharmaceutiquement acceptable.

8. Utilisation d'un composé de formule (I) selon l'une quelconque des revendications 1 à 4 pour la préparation d'un médicament destiné au traitement des troubles de l'appétit, des troubles gastro-intestinaux, des phénomènes inflammatoires, des maladies du système immunitaire, des troubles psychotiques, de la dépendance alcoolique, de la dépendance nicotinique.

## Claims

1. Compound of formula: 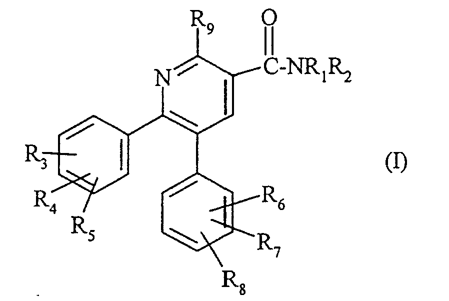 in which:
- R₁ represents hydrogen or a (C₁-C₄)alkyl;
- R₂ represents:
. a saturated mononitrogenous heterocyclic radical containing from 5 to 7 atoms, the nitrogen atom being substituted with an alkanoyl group;
. a group NR₁₀R₁₁;
. a nonaromatic C₃-C₁₀ carbocyclic radical which is substituted more than 3 times with a (C₁-C₄)alkyl group;
. a nonaromatic C₁₁-C₁₂ carbocyclic radical which is unsubstituted or substituted one or more times with a (C₁-C₄) alkyl group;
. a saturated monooxygenated heterocyclic radical containing from 5 to 7 atoms, which is substituted more than 3 times with a (C₁-C₄) alkyl group;
- or R₁ and R₂, together with the nitrogen atom to which they are attached, constitute a piperidin-1-yl radical disubstituted in the 4-position with a phenyl or benzyl group and with a (C₁-C₄)alkyl, (C₁-C₄)alkoxy, cyano, aminocarbonyl, (C₁-C₄)alkylaminocarbonyl, di (C₁-C₄) alkylaminocarbonyl, (C₁-C₃) - alkanoyl or (C₁-C₃)alkanoylamino group; the phenyl or benzyl groups substituting the piperidin-1-yl radical being unsubstituted or substituted with a halogen atom and/or a methyl and/or trifluoromethyl group;
- R₃, R₄, R₅, R₆, R₇ and R₈ each represent, independently of one another, a hydrogen or halogen atom, or a (C₁-C₆)alkyl, (C₁-C₆)alkoxy or trifluoromethyl group;
- R₉ represents a hydrogen atom, a (C₁-C₄) alkyl or cyano group or a CH₂OH, CH₂CN or CH₂O (C₁-C₄) alkyl group;
- R₁₀ and R₁₁, together with the nitrogen atom to which they are attached, constitute a saturated or unsaturated heterocyclic radical containing from 7 to 10 atoms, possibly containing a second hetero atom chosen from O and N, the said radical being unsubstituted or substituted one or more times with a (C₁-C₄)alkyl, hydroxyl, (C₁-C₄)alkoxy or methoxy(C₁-C₂)alkylene group, or substituted with a spirocyclobutane, a spirocyclopentane or a spirocyclohexane;
- or R₁₀ and R₁₁, together with the nitrogen atom to which they are attached, constitute a saturated or unsaturated heterocyclic radical containing 5 or 6 atoms, containing a second nitrogen atom, the said radical being substituted one or more times with a methoxy(C₁-C₂)alkylene group, or substituted with a spirocyclobutane, a spirocyclopentane or a spirocyclohexane;
- or R₁₀ and R₁₁, together with the nitrogen atom to which they are attached, constitute a saturated or unsaturated heterocyclic radical containing 5 or 6 atoms, possibly containing an oxygen atom, the said radical being substituted one or more times with a (C₁-C₄)alkyl, hydroxyl, (C₁-C₄)alkoxy or methoxy(C₁-C₂)alkylene group, or substituted with a spirocyclobutane, a spirocyclopentane or a spirocyclohexane;
in the form of a base or of an addition salt with an acid, and also in hydrate or solvate form.

2. Compound of formula (I) according to Claim 1,
**characterized in that**:
- R₁ represents hydrogen;
- R₂ represents: a group NR₁₀R₁₁;
. a nonaromatic C₁₁-C₁₂ carbocyclic-radical which is unsubstituted or substituted one or more times with a methyl group;
- or R₁ and R₂, together with the nitrogen atom to which they are attached, constitute a piperidin-1-yl radical disubstituted in the 4-position with a phenyl or benzyl group and with a (C₁-C₄)alkyl or (C₁-C₃) alkanoyl group;
- and/or R₃, R₄, R₅, R₆, R₇ and R₈ each represent, independently of one another, a hydrogen or halogen atom, or a (C₁-C₆)alkyl, (C₁-C₆)alkoxy or trifluoromethyl group;
- and/or R₉ represents a hydrogen atom or a methyl, methoxymethylene or cyano group;
- R₁₀ and R₁₁, together with the nitrogen atom to which they are attached, constitute a saturated or unsaturated heterocyclic radical containing from 5 to 10 atoms, possibly containing an oxygen atom, the said radical being unsubstituted or substituted one or more times with a methyl group;
in the form of a base or of an addition salt with an acid, and also in hydrate or solvate form.

3. Compounds of formula (I) according to Claim 1,
**characterized in that**:
- R₁ represents a hydrogen atom;
- and R₂ represents the 3-amino-2,2,5,5-tetramethyltetrahydrofuran group;
- or R₁ and R₂, together with the nitrogen atom to which they are attached, represent a 4-acetyl-4-phenylpiperidin-1-yl group;
- and/or at least one of the substituents R₃, R₄ and R₅ represents a chlorine or bromine atom or a methoxy group;
- and/or at least one of the substituents R₆, R₇ and R₈ represents a chlorine or bromine atom;
- and/or R₉ represents a methyl or methoxymethyl group,
in the form of a base or of an addition salt with an acid, and also in hydrate or solvate form.

4. Compound of formula (I) according to Claim 1, chosen from:
- 1-(1-(6-(4-chlorophenyl)-5-(2,4-dichlorophenyl)-2-methylpyridin-3-yl)-4-phenylpiperidin-4-yl)-ethanone, 6-(4-chlorophenyl)-5-(2,4-dichlorophenyl)-2-(methoxymethyl-N-(2,2,5,5-tetramethyltetrahydrofuran-3-yl)nicotinamide,
- 5-(4-chlorophenyl)-6-(2,4-dichlorophenyl)-2-methyl-N-(2,2,5,5-tetramethyltetrahydrofuran-3-yl)pyridine-3-carboxamide,
- 1-(1-(5-(4-bromophenyl)-6-(2,4-dichlorophenyl)-2-(methylpyridin-3-yl)carbonyl)-4-phenylpiperidin-4-yl)ethanone,
- 6-(4-bromophenyl)-5-(2,4-dichlorophenyl)-2-methyl-N-(2,2,5,5-tetramethyltetrahydrofuran-3-yl)-nicotinamide,
in the form of a base or of an addition salt with an acid, and also in hydrate or solvate form.

5. Process for preparing a compound of formula (I) according to any one of Claims 1 to 4, **characterized in that** a functional derivative of 5,6-diphenyl-3-pyridinecarboxylic acid, of formula: 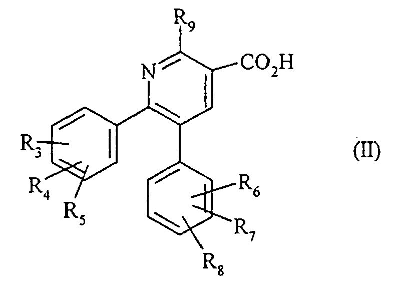 in which R₃, R₄, R₅, R₆, R₇, R₈ and R₉ are as defined for (I) in Claim 1, is treated with an amine of formula HNR₁R₂ (III) in which R₁ and R₂ are as defined for (I) in Claim 1.

6. Medicinal product, **characterized in that** it comprises a compound of formula (I) according to any one of Claims 1 to 4, or an addition salt of this compound with a pharmaceutically acceptable acid, or else a hydrate or a solvate.

7. Pharmaceutical composition, **characterized in that** it comprises a compound of formula (I) according to any one of Claims 1 to 4, or a pharmaceutically acceptable salt, a hydrate or a solvate of this compound, and also at least one pharmaceutically acceptable excipient.

8. Use of a compound of formula (I) according to any one of Claims 1 to 4, for preparing a medicinal product intended for the treatment of appetite disorders, gastrointestinal disorders, inflammatory phenomena, immune system diseases, psychotic disorders, alcohol addiction or nicotine addiction.

## Patentansprüche

1. Verbindung der Formel: 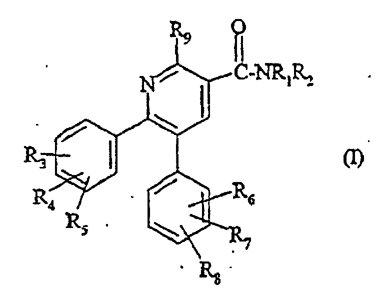 worin:
- R₁ für Wasserstoff oder ein (C₁-C₄)-Alkyl steht;
- R₂ für Folgendes steht:
. einen gesättigten heterocyclischen Monostickstoffrest mit 5 bis 7 Atomen, wobei das Stickstoffatom mit einer Alkanoylgruppe substituiert ist;
. eine Gruppe NR₁₀-R₁₁;
. einen nicht aromatischen, carbocyclischen C₃-C₁₀-Rest, der mehr als 3fach mit einer (C₁-C₄)-Alkylgruppe substituiert ist;
. einen nicht aromatischen, carbocyclischen C₁₁-C₁₂-Rest, der unsubstituiert oder ein- oder mehrfach mit einer (C₁-C₄)-Alkylgruppe substituiert ist;
. einen gesättigten heterocyclischen Monosauerstoffrest mit 5 bis 7 Atomen, der mehr als 3fach mit einer (C₁-C₄)-Alkylgruppe substituiert ist;
- oder R₁ und R₂ zusammen mit dem Stickstoffatom, an das sie gebunden sind, einen Piperidin-1-yl-Rest bilden, der an der Position 4 mit einer Phenyl- oder Benzylgruppe und mit einer (C₁-C₄) -Alkyl- , (C₁-C₄)-Alkoxy-, Cyano-, Aminocarbonyl-, (C₁-C₄)-Alkylaminocarbonyl-, Di-(C₁-C₄)-alkylaminocarbonyl-, (C₁-C₃)-Alkanoyl- oder (C₁-C₃)-Alkanoylaminogruppe disubstituiert ist; wobei die Phenyl- oder Benzylgruppen, die den Piperidin-1-yl-Rest substituieren, unsubstituiert oder mit einem Halogenatom und/oder einer Methyl- und/oder Trifluormethylgruppe substituiert sind;
- R₃, R₄, R₅, R₆ , R₇, R₈ jeweils unabhängig voneinander für ein Wasserstoff- oder Halogenatom, eine (C₁-C₆)-Alkyl-, (C₁-C₆)-Alkoxy oder Trifluormethylgruppe stehen;
- R₉ für ein Wasserstoffatom, eine (C₁-C₄)-Alkyl- oder Cyanogruppe oder eine Gruppe CH₂OH, CH₂CN oder CH₂O(C₁-C₄)-Alkyl steht;
- R₁₀ und R₁₁ zusammen mit dem Stickstoffatom, an das sie gebunden sind, einen gesättigten oder ungesättigten heterocyclischen Rest mit 7 bis 10 Atomen bilden, der ein zweites Heteroatom, ausgewählt aus O und N, enthält oder nicht, wobei der Rest unsubstituiert oder ein- oder mehrmals mit einer (C₁-C₄) -Alkyl-, Hydroxyl-, (C₁-C₄)-Alkoxy- oder Methoxy(C₁-C₂)-alkylengruppe substituiert ist oder mit einem Spirocyclobutan, einem Spirocyclopentan oder einem Spirocyclohexan substituiert ist;
- oder R₁₀ und R₁₁ zusammen mit dem Stickstoffatom, an das sie gebunden sind, einen gesättigten oder ungesättigten heterocyclischen Rest mit 5 bis 6 Atomen bilden, der ein zweites Stickstoffatom enthält, wobei der Rest ein- oder mehrmals mit einer Methoxy(C₁-C₂)-alkylengruppe substituiert ist oder mit einem Spirocyclobutan, einem Spirocyclopentan oder einem Spirocyclohexan substituiert ist;
- oder R₁₀ und R₁₁ zusammen mit dem Stickstoffatom, an das sie gebunden sind, einen gesättigten oder ungesättigten heterocyclischen Rest mit 5 bis 6 Atomen bilden, der ein Sauerstoffatom enthält oder nicht, wobei dieser Rest ein- oder mehrmals mit einer (C₁-C₄)-Alkyl-, Hydroxyl-, (C₁-C₄)-Alkoxy- oder Methoxy(C₁-C₂)-alkylengruppe substituiert ist oder mit einem Spirocyclobutan, einem Spirocyclopentan oder einem Spirocyclohexan substituiert ist;
im Zustand einer Base oder eines Säureadditionssalzes sowie im Zustand eines Hydrates oder Solvates.

2. Verbindung der Formel (I) nach Anspruch 1, **dadurch gekennzeichnet, dass**:
- R₁ für Wasserstoff steht;
- R₂ für: . eine Gruppe NR₁₀-R₁₁;
. einen nicht aromatischen, carbocyclischen C₁₁-C₁₂-Rest, der unsubstituiert oder ein- oder mehrfach mit einer Methylgruppe substituiert ist, steht;
- oder R₁ und R₂ zusammen mit dem Stickstoffatom, an das sie gebunden sind, einen Piperidin-1-yl-Rest bilden, der an der Position 4 mit einer Phenyl- oder Benzylgruppe und mit einer (C₁-C₄)-Alkyl- oder (C₁-C₃)-Alkanoylgruppe disubstituiert ist;
- und/oder R₃, R₄, R₅, R₆, R₇, R₈ jeweils unabhängig voneinander für ein Wasserstoff- oder Halogenatom, eine (C₁-C₆) -Alkyl-, (C₁-C₆)-Alkoxy- oder Trifluormethylgruppe stehen;
- und/oder R₉ für ein Wasserstoffatom oder eine Methyl-, Methoxymethylen- oder Cyanogruppe steht;
- R₁₀ und R₁₁ zusammen mit dem Stickstoffatom, an das sie gebunden sind, einen gesättigten oder ungesättigten heterocyclischen Rest mit 5 bis 10 Atomen bilden, der ein zweites Sauerstoffatom enthält oder nicht, wobei der Rest unsubstituiert oder ein- oder mehrmals mit einer Methylgruppe substituiert ist;
im Zustand einer Base oder eines Säureadditionssalzes sowie im Zustand eines Hydrates oder Solvates.

3. Verbindungen der Formel (I) nach Anspruch 1,
**dadurch gekennzeichnet, dass**:
- R₁ für ein Wasserstoffatom steht;
- und R₂ für die Gruppe 3-Amino-2,2,5,5-tetramethyltetrahydrofuran steht;
- oder R₁ und R₂ zusammen mit dem Stickstoffatom, an das sie gebunden sind, für eine Gruppe 4-Acetyl-4-phenylpiperidin-1-yl stehen;
- und/oder mindestens einer der Substituenten R₃, R₄, R₅ für ein Chlor- oder Bromatom oder eine Methoxygruppe steht;
- und/oder mindestens einer der Substituenten R₆, R₇, R₈ für ein Chlor- oder Bromatom steht;
- und/oder R₉ für eine Methyl- oder Methoxymethylgruppe steht;
im Zustand einer Base oder eines Säureadditionssalzes sowie im Zustand eines Hydrates oder Solvates.

4. Verbindung der Formel (I) nach Anspruch 1, ausgewählt aus:
- 1-(1-(6-(4-Chlorphenyl)-5-(2,4-dichlorphenyl)-2-methylpyridin-3-yl)-4-phenylpiperidin-4-yl)-ethanon, 6-(4-Chlorphenyl)-5-(2,4-dichlorphenyl)-2-(methoxymethyl-N-(2,2,5,5-tetramethyltetrahydrofuran-3-yl)nicotinamid,
- 5-(4-Chlorphenyl)-6-(2,4-dichlorphenyl)-2-methyl-N-(2,2,5,5-tetramethyltetrahydrofuran-3-yl)-pyridin-3-carboxamid,
- 1-(1-(5-(4-Bromphenyl)-6-(2,4-dichlorphenyl)-2-(methylpyridin-3-yl)carbonyl)-4-phenylpiperidin-4-yl)ethanon,
- 6-(4-Bromphenyl)-5-(2,4-dichlorphenyl)-2-methyl-N-(2,2,5,5-tetramethyltetrahydrofuran-3-yl)-nicotinamid)
im Zustand einer Base oder eines Säureadditionssalzes sowie im Zustand eines Hydrates oder Solvates.

5. Verfahren zur Herstellung einer Verbindung der Formel (I) nach einem der Ansprüche 1 bis 4, **dadurch gekennzeichnet, dass** man ein funktionelles Derivat der 5,6-Diphenyl-3-pyridincarbonsäure der Formel: 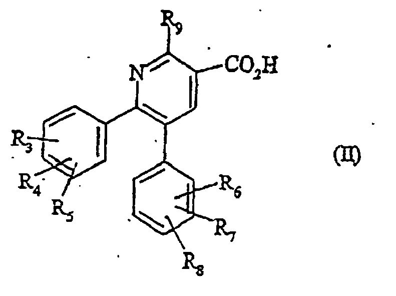 worin R₃, R₄, R₅, R₆, R₇, R₈ und R₉ wie für (I) im Anspruch 1 definiert sind, mit einem Amin der Formel HNR₁R₂ (III), worin R₁ und R₂ wie für (I) im Anspruch 1 definiert sind, behandelt.

6. Arzneimittel, **dadurch gekennzeichnet, dass** es eine Verbindung der Formel (I) nach einem der Ansprüche 1 bis 4 oder ein Additionssalz dieser Verbindung mit einer pharmazeutisch annehmbaren Säure oder auch ein Hydrat oder ein Solvat umfasst.

7. Pharmazeutische Zusammensetzung, **dadurch gekennzeichnet, dass** sie eine Verbindung der Formel (I) nach einem der Ansprüche 1 bis 4 oder ein pharmazeutisch annehmbares Additionssalz, ein Hydrat oder ein Solvat dieser Verbindung sowie mindestens einen pharmazeutisch annehmbaren Exzipienten umfasst.

8. Verwendung einer Verbindung der Formel (I) nach einem der Ansprüche 1 bis 4 zur Herstellung eines Arzneimittels zur Behandlung von Essstörungen, gastrointestinalen Störungen, Entzündungsphänomenen, Erkrankungen des Immunsystems, psychotischen Störungen, Alkoholabhängigkeit, Nikotinabhängigkeit.
